# EUROPEAN PATENT SPECIFICATION

(11) **EP 4 031 877 B1**
(45) Date of publication and mention of the grant of the patent: **18.06.2025**
(21) Application number: 20785871.3
(22) Date of filing: 15.09.2020
(51) Int. Cl.: G01N 33/68, G01N 33/50, C12Q 1/02, C12Q 1/24, B01D 15/08

(54) **IMPROVED NANOLITER-SCALE SAMPLE PROCESSING AND MASS SPECTROMETRY ACQUISITION METHOD FOR SINGLE CELL PROTEOMICS**
VERBESSERTE PROBENVERARBEITUNG IM NANOLITER-MASSSTAB UND MASSENSPEKTROMETRIE-ERFASSUNGSVERFAHREN FÜR DIE EINZELZELL-PROTEOMIK
AMÉLIORATION DU TRAITEMENT D'ÉCHANTILLONS À L'ÉCHELLE NANOLITRE ET MÉTHODE D'ACQUISITION PAR SPECTROMÉTRIE DE MASSE POUR LA PROTÉOMIQUE UNICELLULAIRE

(30) Priority: 16.09.2019 US 201962901022 P
(43) Date of publication of application: 27.07.2022
(73) Proprietor: Battelle Memorial Institute, Richland, Washington 99352 (US)
(72) Inventor: ZHU, Ying, Richland Washington 99352 (US); TSAI, Chia-Feng, Richland Washington 99354 (US); LIU, Tao, Richland Washington 99352 (US); ANSONG, Charles K., Pasco Washington 99301 (US); CLAIR, Geremy CD, West Richland Washington 99353 (US)
(74) Representative: Murgitroyd & Company
(86) International application number: PCT/US2020/050874
(87) International publication number: WO 2022/060345

(56) References cited:
- WO-A1-2018/085835
- WO-A1-2018/170981
- US-A1- 2019 219 592
- US-A1- 2020 033 361
- CHIA-FENG TSAI ET AL: "An Improved Boosting to Amplify Signal with Isobaric Labeling (iBASIL) Strategy for Precise Quantitative Single-cell Proteomics", MOLECULAR & CELLULAR PROTEOMICS, vol. 19, no. 5, 3 March 2020 (2020-03-03), US, pages 828 - 838, XP055742401, ISSN: 1535-9476, DOI: 10.1074/mcp.RA119.001857
- K. MOTAMEDCHABOKI ET AL: "High-throughput...", 31 December 2019 (2019-12-31), XP055742405, Retrieved from the Internet <URL:https://assets.thermofisher.com/TFS-Assets/CMD/posters/po-72483-lc-ms-single-cell-proteomics-hupo2019-po72483-en.pdf> [retrieved on 20201021]
- MAOWEI DOU ET AL: "High-Throughput Single Cell Proteomics Enabled by Multiplex Isobaric Labeling in a Nanodroplet Sample Preparation Platform", ANALYTICAL CHEMISTRY, vol. 91, no. 20, 11 September 2019 (2019-09-11), pages 13119 - 13127, XP055742422, ISSN: 0003-2700, DOI: 10.1021/acs.analchem.9b03349

## Description

### ACKNOWLEDGEMENT OF GOVERNMENT SUPPORT

This invention was made with Government support under Contract DE-AC0576RL01830 awarded by the U.S. Department of Energy as well as NIH Grant No. is CA210955. The Government has certain rights in the invention.

### CLAIM TO PRIORITY

This application claims priority from provisional patent application number 62/901,022 entitled NANOLITER-SCALE SAMPLE PROCESSING AND MASS SPECTROMETRY ACQUISITION METHOD FOR SINGLE CELL PROTEOMICS filed by the same inventors on September 16, 2019 and patent application number 17/021,319, entitled IMPROVED NANOLITER-SCALE SAMPLE PROCESSING AND MASS SPECTROMETRY ACQUISITION METHOD FOR SINGLE CELL PROTEOMICS by the same inventors on September 15, 2020.

### BACKGROUND OF THE INVENTION

Multicellular organisms contain diverse cell types and subtypes with distinct functions. Understanding the heterogeneity or cell subpopulations at single cell level is of great interest for biomedical research. Single cell analysis provides potential insights for understanding unique features of cell populations, cellular lineage, function, differentiation, impacts of microenvironments, and rare cell types in a complex cellular system. Most of current single cell analysis are based on DNA or RNA sequencing, because of the available gene amplification methods like PCR. However, RNA measurements provide an incomplete picture of the molecular state of a cell as proteins mediate the bulk of cellular function. As there is no whole proteome amplification method available, measurements largely rely on the effectiveness of the workflow, as well as the overall sensitivity of instrumental platform.

M. Dou et al (2019) describes high-throughput single cell proteomics enabled by multiplex isobaric labelling in a nanodroplet sample preparation platform. WO 2018/085835 A1 provides methods and systems for biochemical analysis of small cell populations and biological samples. US 2019/0219592 A1 provides mass spectrometry techniques for single cell proteomics. WO 2018/170981 A1 and US 2020/0033361 A1 provide a detection and quantification method for proteomics of post-translational modifications. Researchers at Pacific Northwest National Laboratory recently developed a microfluidic nanodroplet sample preparation approach, termed nanoPOTS (Nanodroplet Processing in One-pot for Trace Samples), that enables single cell proteomic sample processing and analysis using ultrasensitive nanoLC-MS/MS. While this initial workflow yielded good single cell proteome coverage, the analytical throughput was relatively low at ~8 single cells per day. Higher throughput is needed to facilitate efficient analysis of large populations of single cells to provide insights into cellular heterogeneity. The present disclosure provides descriptions of advances in this regard.

Additional advantages and novel features of the present disclosure will be set forth as follows and will be readily apparent from the descriptions and demonstrations set forth herein. Accordingly, the following descriptions of the present disclosure should be seen as illustrative of the disclosure and not as limiting in any way.

### SUMMARY

To address these concerns improved methods of performing proteomic analysis have been developed. Accordingly, the invention provides a method of performing proteomic analysis as defined in independent claim 1. Optional features of the invention are set out in the dependent claims.

In some instances, the method also includes washing the samples from the nanowell using a wash solution and combining the nanowell wash solution into the booster well prior to performing LC separation step. In some instances, the method may further include stabilizing the extracted peptides, which may take place utilizing a number of methods including disulfide reduction. In other embodiments the stabilization step can include alkylation of sulfhydryl groups. Preferably the separating step is performed by liquid chromatography (LC). The step of acquiring sample peptide characterization data may be performed using a mass spectrometer. In some embodiments the chip includes a plurality of nanowells and samples from a number of nanowells are mixed with the booster sample.

In another arrangement a method for performing proteomic analysis is described wherein a single cell sample is placed into each of a plurality of nanowells disposed within a chip. The chip also defining a booster well having a volume greater than any single nanowell, a single cell channel and a booster channel well, the booster well containing a booster sample of known peptides. The method then follows by lysing the cell located in the nanowell, extracting proteins from the cell to form a nanosample, reducing the disulfide groups in the nanosample, alkylating the sulfhydryl groups in the nanosample, performing a digestion on the nanosample, performing TMT labeling on the nanosample, acidifying the nanosample, collecting nanosamples from at least two nanowells and combining these nanosamples with the booster sample to form a mixed sample, and washing the nanowells with a wash solution and adding the used wash solution to the mixed sample. Additionally, the method further includes the step of performing an LC separation to generate a separated sample and may optionally perform MS analysis on the separated sample.

In another example a method of performing proteomic analysis is described wherein single cell samples are placed in a nanowell disposed within a chip. Preferably the nanowell is semispherical and configured to have a diameter < 2 mm and hold liquid volume < 1 µL. A peptide mixture is then placed into a booster well preferably of a similar shape, that is also disposed within the chip. The booster configured to have a diameter > 1 mm and hold liquid volume > 1 µL. lysing the cell sample. The method then follows wherein proteins are extracted from the cell sample, proteins are then disulfide reduced and alkylated, the proteins are digested into peptides, the peptides and the peptide mixture are labeled using tandem mass tag (TMT) labels. The labeled peptides are then collected from the nanowells and collected with the peptides in the booster wells to form a mixed sample. In some instances, this combining all takes place in the booster wells.

Once combined the samples are separated (preferably using an LC column) and data is acquired preferably using a mass spectrometers. The effectiveness of the mass spectrometry data capture can be further enhanced by a variety of methods including: setting the automatic gain control levels on the mass spectrometer during MS2 or MS3 data collection > 5E5; setting injection times during MS2 or MS3 data collection > 250 ms; adjusting the automatic gain control levels and injection times based on the booster/sample ratios and the protein abundance in single cells. In addition, to these steps the separation method also includes pre-fractionating the mixed sample into multiple fractions, such a pre-fractionation step may be performed on a nanoflow high pH liquid chromatography. The fraction may be collected into a low volume container containing dilution buffer < 25 µL. (such a container may include a protein-low-binding material selected from the groups consisting of polypropylene, polyethylene, epoxy, polyetheretherketone, and surface-modified glass). The dilution buffer contains at least one non-ionic and MS-compatible surfactants selected from the group consisting of n-Dodecyl β-D-maltoside, Triton X-100, Tween-20, Tween-80, and NP-40. This method may also include the step of washing the samples from the nanowell using a wash solution and combining the nanowell wash solution into the booster well prior to performing LC separation step. Stabilizing of the extracted peptides may also be performed.

The purpose of the foregoing abstract is to enable the United States Patent and Trademark Office and the public generally, especially the scientists, engineers, and practitioners in the art who are not familiar with patent or legal terms or phraseology, to determine quickly from a cursory inspection the nature and essence of the technical disclosure of the application. The abstract is neither intended to define the disclosure of the application, which is measured by the claims, nor is it intended to be limiting as to the scope of the disclosure in any way.

Various advantages and novel features of the present disclosure are described herein and will become further readily apparent to those skilled in this art from the following detailed description. In the preceding and following descriptions, I have shown and described only the preferred embodiment of the disclosure, by way of illustration of the best mode contemplated for carrying out the disclosure. Accordingly, the drawings and description of the preferred embodiment set forth hereafter are to be regarded as illustrative in nature, and not as restrictive.

### BRIEF DESCRIPTION OF THE DRAWINGS

Figure 1. Shows the workflow of the nanoPOTS-TMT-based single cell proteomics platform and the features and device utilized therein.
Figures 2A-2C show the effects of boosting ratios (0, 25 ×, and 250 ×) on the quantification performance of single cell proteomics. Figure 2A shows the total number of identified peptides. Figure 2B shows Total number of identified proteins; Figure 2C shows Unsupervised PCA showing cell grouping based on protein expressions in single cultured murine cells (C10, Raw, and SVEC).

### DETAILED DESCRIPTION

The following description includes a preferred best mode of one embodiment of the present disclosure.

The following paragraphs describe an embodiment wherein microfluidic nanodroplet technology and tandem mass tag (TMT) isobaric labeling were combined to significantly improve analysis throughput and proteome coverage for single mammalian cells. Isobaric labeling facilitated multiplex analysis of single cell-sized protein quantities to a depth of ~1,600 proteins with median CV of 10.9% and correlation coefficient of 0.98. To demonstrate in-depth high throughput single cell analysis, the platform was applied to measure protein expression in 72 single cells from three murine cell populations (epithelial, immune, and endothelial cells) in <2 days instrument time with over 2,300 proteins identified. Principal component analysis grouped the single cells into three distinct populations based on protein expression with each population characterized by well-known cell-type specific markers. This platform enables high throughput and unbiased characterization of single cell heterogeneity at the proteome level.

Multicellular organisms contain diverse cell types and subtypes with distinct functions. Understanding the heterogeneity or cell subpopulations at single cell level is of great interest for biomedical research. Single cell analysis provides potential insights for understanding unique features of cell populations, cellular lineage, function, differentiation, impacts of microenvironments, and rare cell types in a complex cellular system. Single cell RNA-seq assays have been widely employed to characterize gene expression patterns. However, RNA measurements provide an incomplete picture of the molecular state of a cell as proteins mediate the bulk of cellular function. A compelling body of literature has shown the correlation between RNA and protein abundance is at best moderate. Profiling the proteome (entire complement of the proteins expressed within a cell at a given time) at single cell level is recognized as a substantial technical challenge. As there is no whole proteome amplification method available, measurements largely rely on the effectiveness of the workflow, as well as the overall sensitivity of instrumental platform.

Currently, protein profiling in single cells mainly utilizes targeted, antibody-based approaches, such as Mass Cytometry (CyTOF), Proseek Multiplex, and single-cell western blots. However, these approaches share common shortcomings in that only a limited number of proteins (less than 100 targeted proteins) can be analyzed simultaneously, and the quantification accuracy is largely determined by the specificity of available antibodies. Mass spectrometry (MS)-based proteomic analyses are capable of quantifying thousands of proteins in an unbiased manner. However, due to insufficient sensitivity, large sample losses during preparation, and low throughput, the extension of MS-based proteomics to single cell studies remains challenging. While MS sensitivity has significantly increased with the development of nanoflow separations, electrospray ionization, and more sensitive mass spectrometers, approaches to efficiently process single cells and deliver the sample to MS with minimal sample loss have been largely ineffective. Great effort has been put to improve sample recovery including the use of low-binding tubes, MS- compatible surfactants, immobilized enzyme reactor (IMER)-based approaches, and the development of single-pot workflows such as e.g. the Oil-Air-Droplet chip, and on-valve high temperature trypsin digestion.

Researchers at Pacific Northwest National Laboratory recently developed a microfluidic nanodroplet sample preparation approach, termed nanoPOTS (Nanodroplet Processing in One-pot for Trace Samples), that enables single cell proteomic sample processing and analysis using ultrasensitive nanoLC-MS/MS. NanoPOTS reduces the sample processing volume to less than 200 nanoliters (nL) and the total exposure surfaces to ~1 mm², thus significantly limiting surface losses during sample processing, which is critical for single cell proteomics. Using the nanoPOTS platform, an average of 670 protein groups were confidently identified from single HeLa cells and 160 proteins from a single spiked circulating tumor cells isolated from whole blood. While the current nanoPOTS workflow yielded good single cell proteome coverage, the analytical throughput was relatively low at ~8 single cells per day. Higher throughput is needed to facilitate efficient analysis of large populations of single cells to provide insights into cellular heterogeneity.

Tandem mass tag (TMT) isobaric labeling is an approach developed for multiplexed identification and quantification of proteins from multiple different samples in a single LC- MS analysis/run. Individual samples are first labeled with discrete TMT reagents (also referred to as TMT channels) and then combined into a multiplexed set prior to analysis by high-resolution LC-MS. The proteins in each sample can be identified and quantified based on the TMT reporter ions. Up to 11 samples can be simultaneously measured in single LC- MS runs using TMT 11plex, providing an attractive potential approach/strategy to significantly enhance the throughput of single cell proteomics platform. This concept was initially demonstrated in the proteomic study of single blastomeres isolated from *Xenopus laevis* embryos, where three blastomeres were quantified in single MS run.

Recently, several TMT-based approaches have been developed that facilitate quantification of low abundance proteins. The high-level proteins in tissues facilitated the triggering of MS/MS fragmentation to enable quantifying of low- level proteins in body fluids. Budnik *et al* developed a single cell analysis method (SCoPE-MS) by implementing isobaric labeling of single cells in concert with 200 cells as carrier. Tian *et al.* developed a deep proteomic analysis approach to discover single amino acid variants in a small numbers of cancer cells. Focusing on post-translational modification analysis, Yi *et al.* developed a boosting to amplify signal with isobaric labeling (BASIL) strategy and applied it to study phosphoproteomic dynamics in mass- limited clinical specimens. Common to all these studies, a "boosting" sample containing much larger amount (30-500 fold) of peptide mass was labeled with one TMT channel and the mass-limited study samples were labeled with the remaining TMT channels of the multiplex set. The more abundant boosting peptides provided rich fragment information for peptide identification, while reporter ions provided quantification information for study samples. While these TMT-based boosting methods establish the feasibility of TMT labeling for proteomics analyses of mass-limited samples down to single cells, they uniformly suffer from the drawbacks of minute samples being processed conventionally. Specifically, the attendant sample loses due to non-specific adsorption/adhesion on surfaces.

To address this challenge, we combined the nanoPOTS approach with TMT isobaric labeling method, which was previously used in SCoPE-MS workflow, to improve both proteomic sample processing efficiency and analysis throughput for single cells. Single cells were isolated by flow cytometry, processed in nanowells, labeled with TMT 10-plex, and finally combined for LC/MS analysis. We systematically investigated the labeling reproducibility and the effect of boosting ratios on single cell quantification. The optimized system was applied to measure protein expression in 72 single cells from three cultured murine cell populations (epithelial, immune, and endothelial cells). We demonstrated our single cell proteomics approach provides deep proteome coverage for single cell analysis, can discriminate mammalian cell types and identify cell-specific protein markers.

In one set of experiments, we employed the BD Influx II cell sorter (BD Biosciences, San Jose, CA) to directly isolate single cells into nanowells. A customized matrix was generated in the cell sorter control software to match the nanowell array design. Fluorescent beads were used to optimize sorting parameters and to confirm successful deposition in each well. To select only viable cells, murine cells were labeled with a membrane-permeable live-cell labeling dye (100 nM, Calcein AM, eBioscience, Thermo Fisher) for 30 min in the dark. After cell collection, the nanowell chips were stored at -80°C or directly submitted for proteomic sample preparation.

In use the nanowell chip was placed in a closed chamber that was maintained at 95% relative humidity during dispensing procedures to minimize liquid evaporation in nanowells. The nanoPOTS-based sample preparation was carried out as the following steps. (1) Cell lysis, protein extraction, and disulfide reduction: 100 nL solution containing 0.2% DDM and 5 mM TCEP in 100 mM TEAB was added into each of the nanowells and the nanowell chip was incubated at 70 °C for 30 min. (2) Alkylation of sulfhydryl groups: 50 nL 30 mM IAA in 100 mM TEAB was dispensed into each nanowell and the nanowell chip was incubated at room temperature in dark for 30 min. (3) Lys-C digestion: 50 nL Lys-C solution containing 0.5 ng Lys-C in 100 mM TEAB was added into each nanowell and the nanowell chip was incubated at 37 °C for 4 h. (4) Tryptic digestion: 50 nL trypsin solution containing 0.5 ng trypsin in 100 mM TEAB was added into each nanowell and the nanowell chip was incubated at 37 °C for 6 h. (5) TMT labeling: each of the 100 nL dissolved TMT reagents (0.8 mg in 41 µL acetonitrile) was added into the corresponding nanowells; for the boosting channel well where relatively large amount of peptides mixture was preloaded, 300 nL of the dissolved TMT-131 (0.8 mg in 41 µL acetonitrile) was added; the nanowell chip was incubated at room temperature for 1 h. (6) TMT quenching: 50 nL and 300 nL of 2% hydroxylamine solution was added into single cell channel and boosting channel wells respectively, and the nanowell chip was incubated at room temperature for 15 min. (7) Acidification: 50 nL 5% formic acid solution was added into nanowells and the nanowell chip was incubated at room temperature for 15 min. (8) Sample collection: the TMT labeled peptides from multiple nanowells were collected and combined into the booster well and mixed with boosting peptides. We also performed one round of nanowell wash to increase the recovery and the wash solution was also combined into the booster well. The combined samples were stored in a section of fused silica capillary (200 µm i.d., 360 µm o.d.) with both ends sealed with Parafilm. The capillary section was stored at -20 °C prior to nanoLC-MS/MS analysis.

Both SPE precolumn (100 µm i.d., 360 µm o.d., 4 cm long) and LC column (30 µm i.d., 360 o.d., 50 cm long) were slurry-packed in house with 3-µm C18 packing material (300 Angstrom pore size Phenomenex, Terrance California. The sample storage capillary was connected to the SPE precolumn using a PEEK union (Valco instruments, Houston, TX). Sample was loaded and cleaned up in the SPE precolumn using a nanoACQUITY UPLC pump (Waters, Milford, CT) by infusing Buffer A (0.1% formic acid in water) at a flow rate of 1000 nL/min for 10 min. The SPE precolumn was then reconnected to the LC column using a low-dead-volume PEEK union (Valco instruments, Houston, TX) for LC separation. The flow rate of the LC separation was 50 nL/min with the split from 300 nL/min using a nanoUPLC pump (Dionex UltiMate NCP-3200RS, Thermo Scientific, Waltham, MA). A linear 100-min gradient of 8-30% Buffer B (0.1% formic acid in acetonitrile) was used for the LC separation. Then the LC column was washed by ramping Buffer B to 45% in 15 min and 90% in 5 min, and finally re-equilibrated with 2% Buffer B for another 10 min.

The separated peptides were ionized at a spray voltage of 2 kV and the ions were collected into an ion transfer capillary set at 150°C. The RF lens was set at 30%. The MS1 scan was set at a mass range from 375 to 1575, a scan resolution of 120 k, an AGC target of 1E6, and a maximum injection time of 50 ms. Precursor ions with charges of +2 to +7 and intensities >20,000 were selected for MS/MS sequencing. Precursor ions were isolated with an m/z window of 0.7 Th and fragmented by high energy dissociation (HCD) set at 35%. Repeat sampling were reduced with an exclusion duration of 60 s and m/z tolerance of ±10 ppm. MS/MS scan was carried out in the Orbitrap with an AGC target of 1E5. The MS/MS scan resolutions and maximum injection times were set as 60 k and 246 ms.

The spectral identifications were performed using MS-GF+ on the Thermo RAW files. Searches were performed in semi-tryptic mode with a tolerance set at 20 ppm, Methionine oxidation was set as variable modification (+15.9949 Da), TMT 10 plex (+229.1629 Da) and cysteine carbamidomethylation (+57.0215 Da) were set as fixed modifications. The maximum peptide length was set to be comprised at a minimum of 6 and a maximum of 50 residues. Only ions with precursor charge comprised between 2 and 5 were considered for the search. For the HeLa digest, the human UniProt KB database (fasta file downloaded 2017-04-12, 20,198 sequences) was used for the search; for all the other experiments, the murine UniProt KB database (fasta file downloaded 2017-04-12, 16,865 sequences) was used for the search, in both case a self-assembled list of human, bovine and porcine usual contaminants (16 sequences) was added to the database searched. MS-GF+ creates its own decoy database from the searched fasta files to enable to estimate the False Discovery Rates (FDR). The TMT10plex reporter intensities were extracted using MASIC v3.0.7111 (https://github.com/PNNL-Comp-Mass-Spec/MASIC/releases/tag/v3.0.7111) with a reporter tolerance of 0.003 Da. Internally, all the data was managed through the PRISM Data Management System.

The subsequent data processing was performed in R v3.5.1 using the package RomicsProcessor v0.1.0, which is available on Github (https://github.com/PNNL-Comp-Mass-Spec/RomicsProcessor) and Zenodo (https://zenodo.org/record/3386527). This package that comprise several modules enabling to perform data assembly, data visualization, data clustering, allows to perform data subsetting, various normalization methods and general statistics. This omics-oriented package enables to process data in a non-destructive fashion by saving the original data frame, its associated metadata, the transformative steps of the data processing and the processed data in the same multilayered type of R object named "romics_object". Additionally, a data processing folder was created for each data analysis performed. Each data processing folder contains the file generated by MS-GF+ and MASIC, the corresponding metadata, an annotated R markdown notebook, an html report containing the versions of all the packages utilized, all the figures generated in pdf format and the data at different points of the processing (i.e. after first assembly and at the end of the normalization steps).

The general data normalization and interpretation pipeline included the following steps described below. The identified spectra (MS-GF+ table) were merged with their reporter intensities (MASIC table); a False Discovery Rate (FDR) filtering was applied in order to obtain an FDR below 1% at the spectra, PSM and protein levels (only the proteins identified with at least two unique PSMs were conserved); an isotope impurity correction was applied; the data was rolled up to the PSM and protein level (this type of normalization has the advantage of putting less weight in low abundant peptides); The resulting PSM and protein data frames were then log2 transformed; the method implemented in the package pmartR v0.9.0 was used to filter out technical or process-based and extreme biological outliers; after a median centering of the data, for the large single cell dataset, only the PSMs or Proteins with a maximum missingness of 40% within at least given single cell-type were conserved for quantification. Finally, a ComBat batch correction from the SVA package v3.30.0 was applied to remove the obvious TMT-set specific batch effects. The evaluation of the data was performed at multiple steps of the data analysis process using diverse metrics including the distribution of the Coefficient of Variation, the correlation values when working with similar samples, hierarchical clustering, and Principal Component Analysis (PCA). As PCA requires full data, the missing values were imputed using the method implemented in the package missMDA v1.14 and the analysis was performed using the Package FactoMineR v1.41; the missingness of the data was restored prior to export the resulting and to perform pairwise two-tailed heteroscedastic student T.tests. Only the proteins with higher abundance in one condition compared to the two others (T.test p<0.05) were considered enriched in the corresponding cell type.

To enable the TMT labeling and boosting concept into the nanoPOTS technology, we re-designed the layout of the first generation nanoPOT chips. In this embodiment a series of smaller and larger diameter nanowells are patterned onto the nanoPOTS chip. The smaller diameter (1.2 mm) nanowells are utilized to hold single cells and the large diameter (1.8 mm) nanowells are utilized for boosting samples, as well as for sample combination (or multiplexing) within the same TMT set. The total surface area of the smaller diameter nanowells utilized to hold single cells or single cell-sized protein quantities is ~1 mm². This is a much smaller effective total surface area compared to the conventional 0.5-mL tubes (-130 mm²) utilized in other single cell and/or mass-limited analyses described, providing the benefit of significantly reduced opportunity for surface-adsorption- induced protein/peptide (i.e. sample) loss and thus presumably higher sensitivity.

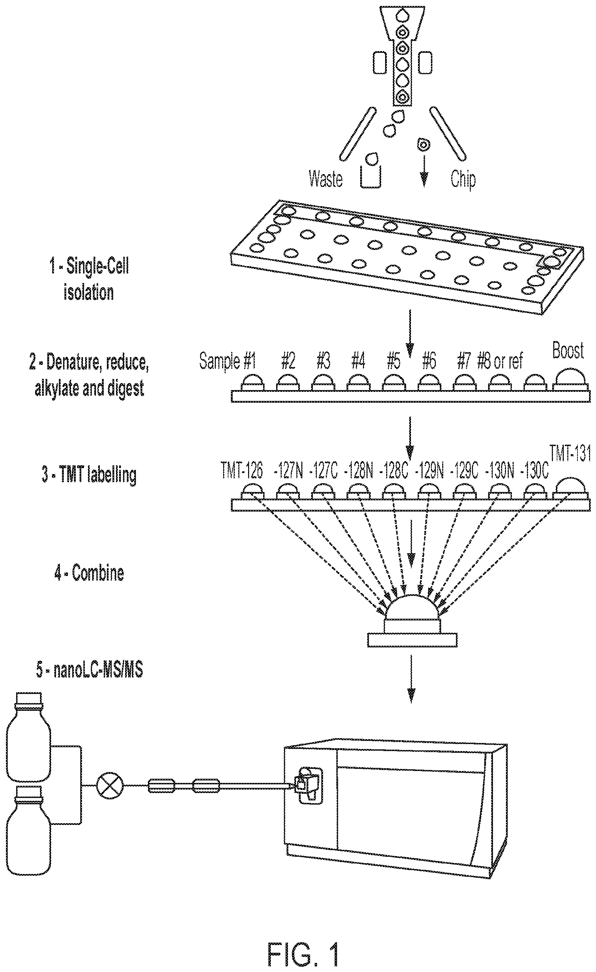

Figure 1 shows the integrated workflow of nanoPOTS processing and TMT-labeling on the new chip design. In one embodiment, cells or protein lysate are deposited into nanowells and protein extraction (as needed), reduction, alkylation and digestion are performed as previously described. 100 nL (for smaller nanowell) or 300 nL (for larger nanowell) of dissolved TMT reagents are then added to the appropriate nanowells. When using TMT10plex and implementing the boosting concept, up to 9 samples (including the boosting sample) can be combined (or multiplexed) and analyzed in a single LC-MS run as depicted in Figure 1. Here the boosting sample is labeled with the TMT 131 channel and the 130N channel is left empty due to isotopic contamination from the 131 channel. To maximize overall sensitivity of LC-MS analysis, the combined samples (i.e. multiplexed TMT set) are analyzed using a 30-µm-i.d. nanoLC column operated at 50 nL/min with an Orbitrap Fusion Lumos mass spectrometer. Given a typical 120-min LC gradient and 8 samples of interest (e.g. single cells) multiplexed in a single TMT10plex set, the nanoPOTS-TMT platform can analyze ~70 samples (e.g. single cells) per day, or over ~490 samples (e.g. single cells) per week.

In traditional TMT-based quantitative proteomics, TMT-labeling is typically done with samples at ng-µg levels. However, single mammalian cells only contain sub-ng levels of protein (0.1 to 0.5 ng total protein), thus it is necessary to assess whether the TMT-labeling strategy will provide high quantification accuracy at this scale. We evaluated TMT- labeling of single cell-level protein material in our nanodroplet processing platform (nanoPOTS) using commercially available HeLa protein digest from Thermo Scientific. We reasoned that a commercially available and quality-controlled digest will more readily facilitate future benchmarking efforts by the scientific community, a current challenge. Single-cell-equivalent amounts (0.2 ng) of the HeLa digest were dispensed into seven of the small diameter nanowells and 10 ng of HeLa digest was dispensed into a larger nanowell. The single-cell-equivalent samples were then labelled with the first seven channels of the TMT10plex reagent (i.e. from 126 to 129N) and the larger (10 ng) boosting sample was labelled with the 131 channel.

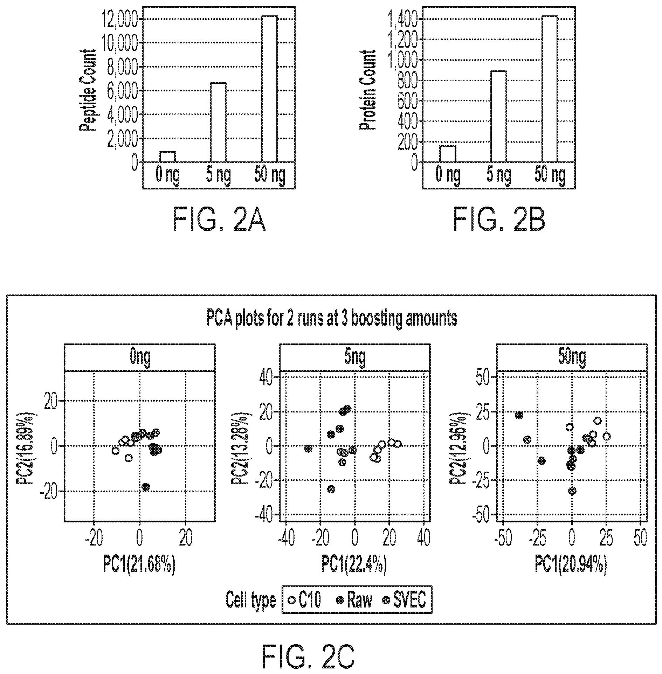

We first evaluated the TMT labeling efficiency by comparing the number of unlabeled peptides with that of labeled peptides. The labeling efficiency was ~99% for each of the duplicate LC-MS analyses, indicating that our nanoPOTS platform provides a highly efficient TMT labeling reaction at the nanoliter-scale and for the sub-ng protein amounts. The median values of log2-transformed protein intensities from each of the 0.2-ng HeLa digests ranged from 14 to 14.4, and for the 130C and 130N empty channels the log2-transformed median intensity was 9.9 and 10.8, respectively (Figure 2A and Figure S2A). The intensity values observed for the empty channels arise from a combination of isotope contamination and chemical noise, two well recognized phenomena. Pair-wise analysis of the single-cell-equivalent TMT channels showed Pearson's correlation coefficients ranging from 0.97 to 0.99. The median coefficient of variation (CV) of the seven single-cell-equivalent samples in each of the two multiplexed TMT sets analyzed by LC-MS was ~11%.

When the LC-MS data from the two multiplexed TMT sets (corresponding to 14 single- cell-equivalent samples) were combined, a higher CV with a median value of 24.1% was observed as anticipated with no batch correction. Principal component analysis (PCA) clearly showed most of the variance resulted from batch artifacts. With the batch covariate identified as being the TMT set run, we employed an empirical Bayes method, using the ComBat function from the R package sva to perform batch correction. After batch correction, the two TMT sets were indistinguishable and the median CV of protein intensities observed in the two combined TMT datasets dropped to ~11%. Across the two datasets 11,988 unique peptides and 1,604 proteins (requiring at least 2 unique peptides per protein) were identified for the single-cell- equivalent (0.2 ng) HeLa digests, with 79% of the peptides and 98% of the proteins common between the two datasets (Figure 2D). Taken together, these results indicate robust high-throughput quantification can be achieved for single cell proteomics by combining isobaric labeling and nanodroplet-based sample processing. In this boosting strategy, the boosting ratio (i.e. ratio between the amount peptide in the boosting channel and in the remaining sample channels) reported in the literature ranges from 30 to 500-fold. However, the impact of boosting ratio (specifically the mass ratio of boosting peptides to single-cell-sized peptides) on quantification accuracy for single cell-level analyses has not been well-characterized and remains unclear. Therefore, we evaluated the effect of boosting ratios on quantification for single cell analyses.

We used TMT-labeled single cells from three cultured murine cell lines (C10, epithelial cells; RAW, macrophage cells; SVEC, endothelial cells). Three levels of boosting material were tested: 0 ng (no boosting), 5 ng and 50 ng. The protein digest used for boosting contained equal amounts of proteins from each of the three cell types. Single mammalian cells are estimated to have an average protein content of ~0.2 ng, thus the 5 ng and 50 ng protein digests correspond to boosting ratios of ~25 and 250, respectively. For each of the three tested boosting ratio scenarios, two independent TMT sets were prepared and analyzed. As expected, both peptide and protein identifications increased with increasing boosting ratios. At 0-fold, 25-fold and 250-fold boosting ratios the number of identified proteins in single cells were 171, 890, and 1408, respectively. A label-free bulk analysis of the proteome of 5 replicates of the same three cell types (C10, RAW, SVEC) employed above revealed that their proteomes were sufficiently distinct to enable distinguishing of the three cell types based on protein expression alone.

We anticipated that the same would hold true at the single cell-level given reproducible and accurate quantification of single cell proteomes. For each boosting ratio scenario described above (i.e. 0-fold, 0 ng; 25-fold, 5 ng; 250-fold, 50 ng), an unsupervised PCA was performed on protein intensities extracted from TMT reporter ions. While individual cells clustered well by type for the samples employing the 0-fold (0 ng) or 25-fold (5 ng) boosting scenario, this was not the case for the samples employing the 250-fold (50 ng) boosting scenario. Interestingly, although only 170 highly abundant proteins were quantified in single cell samples without boosting, the cell types grouped based on their proteome profile alone, indicating the nanoPOTS-TMT- based single cell proteomics platform has good potential to distinguish cell populations without the requirement of additional input. As noted above, while the 250-fold (50 ng) boosting scenario improved proteome coverage compared to the 25-fold (5 ng) boosting scenario, it however yielded reduced cell type specific clustering compared to the 25-fold (5 ng) boosting scenario.

We performed an analysis of the data to understand the factor(s) driving this observation. Examination of the data suggested the level of missing values (i.e. missingness) within a dataset was a main driver of the above observation. When the level of boosting was enhanced from the 25-fold to 250-fold, the level of missingness at the peptide level and protein level for single cell channels increased from 20% to 42% and 3% to 11% respectively. Additionally, the level of unshared peptides and unshared proteins increased from 45% to 55% and from 6% to 11% respectively, with the greater boosting level. We reasoned that the increased missing values caused the reduced single cell quantification performance at the higher 250-fold boosting scenario. Taken together, these results suggest there is an optimum boosting scenario that should ideally be determined for the cell types being studied prior to utilizing the boosting strategy.

To further demonstrate an initial application of our platform for high throughput and deep single cell proteome profiling, we applied it to quantify single cell proteomes from a larger population of cells. 72 single cells, corresponding to 24 C10 cells, 24 RAW cells, and 24 SVEC cells, were deposited onto nanoPOTS chips using FACS as we previously described to perform the large scale single cell experiment. To minimize the incidence of dead cells, cellular debris and precipitates deposited into nanowells of the nanoPOTS chip and to ensure analysis of only viable cells, we utilized Calcein AM, a membrane permeant dye that fluoresces only in metabolically active cells. Isolation and deposition of cells into nanowells of the nanoPOTS chip gated on the Calcein AM signal readily distinguished viable cells from dead cells and other contaminants. The single cells were deposited onto nanoPOTS chips and labeled with discrete TMT channels. A total of 4 nanoPOTS chips were utilized to accommodate the 72 single cells, with each nanoPOTS chip harboring 6 individual single cells distributed evenly across the three cell types.

From LC-MS/MS analyses of the 12 multiplexed TMT batches containing 72 single cells we identified 2,331 proteins at a <1% false discovery rate (FDR) and conservatively 1,597 proteins identified with at least 2 unique peptides at <1% FDR. For quantitative analysis of the single cell proteome data, we utilized a multiparametric method implemented in the pmartR package to identify and exclude outliers from further downstream processing. A total of eleven cells were considered as technical, process-based or extreme biological outliers and were excluded from further analysis. From the remaining 61 single cells only proteins present in 60% of the datasets from a given cell type (C10, RAW, or SVEC cells), equivalent to proteins present in 44% of all datasets across all cell types, were considered for quantification resulting in 1,225 quantifiable proteins. The levels of all 1,225 proteins quantified in single cells were projected onto their principal components. Principal component analysis (PCA) showed the single cell proteomes grouped by cell type.

To identify features driving the grouping of the three cell types we performed pairwise Student's tests (p<0.05) utilizing non-imputed data. Pairwise Student's tests (p<0.05) revealed 138 proteins were enriched in C10 cells compared to RAW and SVEC cells, 82 proteins were enriched in RAW cells compared to C10 and SVEC cells, and 56 proteins were enriched in SVEC cells compared to C10 and RAW cells. Importantly, markers of each of the three cell types (C10 epithelial cells, RAW macrophage cells, SVEC endothelial cells) were present at higher abundance in the expected single cell populations

Ezrin, a protein normally located in the apical membrane of lung epithelial cells was significantly higher in C10 single cells compared to the other two cell types (T.test p<0.001). This observation supported our earlier label-free nanoPOTS-based analysis of human sorted lung epithelial and mesenchymal cells where we also identified ezrin as significantly enriched in lung epithelial cells. Moesin, a paralog of ezrin known to be enriched in the apex of some microvilliated epithelial cells and Collagen I were also enriched in C10 single cells. CD68, CAPG, and LYZ1, three macrophage and/or immune cell markers were all enriched in RAW single cells. CD68, CAPG, and LYZ2, three macrophage and/or immune cell markers were all enriched in RAW single cells. CD68 is a macrophage and monocyte surface marker protein. CAPG is a macrophage-capping protein. LYZ2 is an antimicrobial enzyme that is exclusively expressed in monocytes, macrophages and granulocytes. SVEC4-10 is an endothelial cell line derived by SV40 (strain 4A) transformation of endothelial cells from axillary lymph node vessels with known antigens/markers of this cell line described by ATCC including H2 class I histocompatibility protein (H2-K1), Factor VIII related antigen and VCAM. While we did not detect the latter two proteins in our single cell proteomics dataset, H2- K1 was enriched in single SVEC4-10 cells. Prosaposin, a protein known to be high in lymphocytes and STAT1 described to be most abundant in lymphoid tissues on the Human proteome atlas, were also found enriched in SVEC single cells. These results are largely in agreement with bulk-scale label-free abundance measurements.

The foregoing descriptions and data show that combining the nanoPOTS technology with TMT isobaric labeling to establish a platform that provides a robust and high-throughput proteomic workflow for effective multiplexed analysis of single mammalian cells. We found the high reaction kinetics at nanoliter scale allow to use minimal labeling reagents and thus significantly reduce chemical contamination originating from excessive reagents. Reproducible quantitative proteome measurements across >60 single cells were achieved, with proteome coverage >1,200 proteins when conservatively requiring 2 unique peptides per protein quantified and presence in >40% of the cells analyzed. The enabling aspect of our platform is well demonstrated by the ability to group single cells by cell type based on their protein expression alone. We anticipate that our single cell proteomics platform should enable broad applications involving single cells or very limited starting quantities of material, e.g. understanding stem cell development, proteomic studies of isolated clinical specimens such as circulating tumor cells, or unbiased proteome imaging of tissue heterogeneity at cellular resolution.

While the current single cell proteomics platform provides significantly improved analytical throughput (~490 cells analyzed per week), we note that a new TMT16plex reagent from ThermoFisher coupled with improvements in platform automation and LC- MS gradient optimizations we are implementing is poised to increase current throughput by 3-4-fold. In addition to throughput, the single cell quantification accuracy can be further improved by incorporating gas phase fractionation technologies including high-field asymmetric waveform ion mobility spectrometry (FAIMS) and real-time search MS³ (RTS-MS³). We anticipate these ion fractionation strategies not only efficiently minimize ratio compression and improve proteome dynamic range, but also significantly reduce chemical noise and related false discovery. Finally, we also plan to reduce the sample processing volumes to low nanoliter or even picoliter scale, which could further improve protein recovery of single cells and reduce chemical contamination from reagents.

**In** addition to this process for sample handling the additional use of other techniques have also been useful in driving toward optimized results. As mentioned above, taking advantage of the multiplexed detection enabled by isobaric labeling, as well as boosting/carrier strategies can provide for enhanced sensitivity. Our research has shown that the iBASIL method that can be easily implemented for broad applications. Using BASIL and "BASILlike" approaches, there is always a balance between the achievable proteome coverage and quantification quality for each individual parameter.

One BASIL (Boosting to Amplify Signal with Isobaric Labeling) strategy for enabling comprehensive phosphoproteomic analysis of smaller samples (16) e.g. quantification of 20,000phosphosites from human pancreatic islet) includes boosting-to-sample ratio varied from 30 to 200 or even 500 in different studies. We found that excessively high boosting ratios degrade both signal stabilities and signal-to-noise ratios (S/N) of lower abundance proteins, due mainly to the limited charge capacity of the Orbitrap; significantly increased automatic gain control (AGC) and ion injection time (IT) settings that those used in typical bulk analysis, on the other hand, help improve the signal in the sample channels, resulting in precise protein quantification in the single cells while achieving improved proteome coverage. This improved BASIL (iBASIL) strategy was demonstrated by the reliable precise quantification of 1500 proteins in 104 FACS-isolated single cells, which led to robust separation and clustering of these cells from 3 different acute myeloid leukemia (AML) cell lines.

In particular we found that, a B/S ratio of 100, in conjunction with high AGC and IT values as starting points for iBASIL implementation and optimization appear to provide optimized results. When extremely high B/S ratios (e.g. 1000) are used to increase the proteome coverage, higher AGC and/or IT settings are preferable to achieve reliable quantitation, but with the trade-off of fewer quantifiable proteins. One optimized iBASIL strategy enabled precise quantification of 1500 proteins in 104 FACS-isolated single AML cells by coupling with nanoPOTS-based sample preparation platform. These results allowed for robust clustering and separation of single cells from 3 different AML cell lines and revealed functionally distinct differences in their proteomes. we believe that iBASIL will have broad utility in systems biology and biomedical research for precise quantitative single cell proteomics and nanophosphoproteomics, as well as for analysis of precious mass-limited clinical specimens not readily accessed by current proteomics platforms.

Additional studies showed that the total number of trapped ions is limited by the space charge effect and is in the range of 1 million elementary charges. Because of this limitation, ions from the single-cell samples cannot be effectively detected when high boosting-to-sample ratios (B/S ratios) are used. However, unlike the increased TMT signals in the boosting channel, the TMT signals in the sample channels decreased as the B/S ratios increase resulting in higher standard deviations (S.D.), decreased Pearson correlation coefficients (from 0.991 to 0.967; , and increased median coefficient of variations (CVs) (from 7.7% to 11.9%; ) for the TMT signals from sample channels. Note that the TMT intensities of the empty channels were not affected as the B/S ratios increase. A similar trend was also observed for 10 ng samples of peptides with varying B/S ratios. High B/S ratios provide increased peptide/protein identifications at the expense of quantitation quality.

In addition, testing showed that higher AGC allowed for accumulation of more ions from both study and boosting channels providing significantly increased TMT signals from the study channels, which can be attributed to the dramatically increased ITs. The enhanced TMT signal in the study sample channels led to significantly improved quantitation quality. For example, the Pearson correlation coefficients increased from 0.933 to 0.994, the median CVs decreased from 14.6% to 8.8% and the missing value decreased from 2% to 0.2%. However, the number of quantifiable peptides decreased from 7208 to 5993 when the AGC increased from 5E4 to 5E6 because of the significantly increased duty cycle times which resulted in reduced MS2 scan rate. A similar trend was also observed for 0.5 ng input samples at a B/S ratio of 1000 with varying AGC settings. The higher AGC can greatly improve the quantitation performance particularly when high boosting rations were used.

In addition to AGC, the maximum IT is another parameter that can be modified to control the total number of peptide ions for MS2 analysis. Higher AGC settings allow for accumulation of more ions from the study sample channels with the presence of the boosting sample. In one set experiments, tryptic peptides labeled individually with TMT126, 127N and TMT127C were mixed with the TMT131N-labeled boosting peptides at the B/S ratios of 10, 50, 100, and 200, and analyzed with different ITs ranging from 300 ms to 1,000 ms whereas AGC was fixed at 5E5. Consistent with a previous report that longer ITs can increase MS detection sensitivity for low abundance proteins, the median TMT signals from the sample channels and the Pearson correlation coefficients were improved using longer ITs for all the B/S ratios. Longer ITs also led to increased number of quantifiable peptides at the lowest B/S ratio (10). However, at larger B/S ratios (e.g. 100), the numbers of quantifiable peptides rapidly decreased because of the higher duty cycle times.

The results from the evaluation of B/S ratios and AGC and maximum IT settings clearly show a trade-off between achievable proteome coverage and quality of quantification. Our measurements suggest a B/S ratio of 100 and relatively high AGC (5E5) and IT (300 ms) settings are a reasonable starting point for implementation of iBASIL for single cell analysis. In another set of experiments, the iBASIL settings for quantitative single-cell proteomics analysis using boosting (as compared with the typical bulk analysis MS settings), single cell equivalents (0.1 ng of tryptic peptides) from three AML cell lines (MOLM-14, K562 and CMK) were labeled individually with TMT126-130C and then combined with the TMT131N-labeled boosting sample at a B/S ratio of 1000. The B/S ratio of 1000 was used to represent the worst-case scenario for boosting (larger than those reported in the literature). TMT130N channel was left empty to avoid skewed measurements caused by isotopic impurity of the TMT131N reagent (i.e. the boosting channel).

The samples were analyzed by both the optimized iBASIL MS settings (AGC 5E6; max IT 300 ms) and normal MS settings typically used for conventional bulk analysis (AGC 5E4; max IT 100 ms). When compared with the results obtained using the normal MS setting, iBASIL significantly increased the number of quantifiable peptides from 900 to 3541 and proteins from 585 to 1131. In addition, the median values of TMT signals in the study sample channels increased by 4 fold when the improved iBASIL settings were implemented resulting in better quantification as evidenced by the improved separation and clustering of the AML cells in the PCA analysis. We then evaluated the potential impact of a large B/S ratio (1000) on quantification, even with the iBASIL settings, using the same three AML cell lines at different B/S ratios (no missing value at least in one cell type) and TMT reporter ion intensity for the sample channels for the same samples analyzed with regular BASIL and iBASIL (An improved Boosting to Amplify Signal with Isobaric Labeling (iBASIL) strategy for Precise Quantitative Single-cell Proteomics, Molecular & Cellular Proteomics, 2020, 19, 5, 828). PCA analysis using the quantifiable proteins showed the separation of three AML cell lines using the normal boosting, 100, and 1000.

As expected, the number of quantifiable peptides and proteins increased with the B/S ratio. For all the B/S ratios, the three cell types were reasonably well clustered and separated in the PCA analysis; however, the quality of the separation and reproducibility did slightly decline as the B/S ratio increased. Interestingly, only a moderate increase (14.4%) in the quantifiable proteins was observed when the B/S ratio was increased from 100 to 1000, suggesting that extremely high B/S ratios may not be needed for achieving deep proteome coverage.

The results further showed that at the large B/S ratio of 1000, when TMT131N was used as the boosting channel, in addition to TMT130N, two additional TMT sample channels (TMT129N and TMT130C) were also significantly affected, most likely because of isotopic impurities of TMT131N. Therefore, when higher B/S ratios are used, the experimental design should be modified to leave the affected channels empty (we also note these observations suggest the utility of future modifications to the mass tagging reagents to reduce/eliminate the reduced multiplexing or distorted quantification resulting from reagent isotopic impurity. To further evaluate the quantification at different B/S ratios, we also calculated the fold change between any two cell types and performed pairwise correlation for the data with and without boosting. The fold changes in the data with 100x boosting and without boosting exhibited good correlation with Pearson correlation coefficients of 0.74, 0.83 and 0.87 for the three pairwise comparisons, respectively suggesting an overall good quantification with 100 boosting.

The slopes of linear regression for the same pairwise comparisons, however, were 0.59, 0.79 and 0.84, respectively, which indicates that the dynamic range in quantitation was slightly reduced after applying the boosting strategy; this was further confirmed by the poorer correlation and lower slopes when comparing the data with 1000 boosting and without boosting. These results showed that although the boosting strategy can significantly increase the proteome coverage for single-cell analysis, the higher boosting ratio would reduce the dynamic range in quantitation because of the limited dynamic range of MS detectors, e.g. the linear dynamic range of Orbitrap (intrascan) range from 1000 to 10,000 (26).

Collectively, the data showed that the B/S ratios should be carefully selected for quantitative analysis of small-sized samples with matched AGC and IT settings for well-balanced results in both proteome coverage and quantification quality. Comparing iBASIL-MS2 to iBASIL-MS3 methods in TMT-based quantitative global proteomic analysis, the greater selectivity provided by SPS-MS3 has been shown to mitigate the isobaric labeling ratio "compression" issue associated with MS2 approaches. Finally, the optimized iBASIL strategy was applied to enable precise quantitative proteomic analysis of FACS-sorted single MCF10A cells. Single cells were sorted and processed with the nanoPOTS platform. Ten nanograms of tryptic peptides from a bulk MCF10A cell digest was used as the boosting channel. When compared with single-cell proteomics results without boosting, 2-fold more proteins (from 434 to 853; 390 and 664 have two or more peptides hits, respectively) were quantified by iBASIL. The quantification performance of the iBASIL MS settings (AGC: 5E6;IT: 300 ms) and other reported MS settings for single-cell proteomics analysis (AGC: 5E4; IT: 300 ms) was also compared. Although the number of quantifiable proteins was slightly lower, the sample TMT reporter ion intensities using the iBASIL settings were significantly increased by 1.8-fold. The iBASIL strategy allowed for precise quantification of over 1,500 proteins that reveal functionally distinct differences in the single cells, representing a significant step forward toward comprehensive, high fidelity single-cell proteomics analysis.

While various preferred embodiments of the disclosure are shown and described, it is to be distinctly understood that this disclosure is not limited thereto but may be variously embodied to practice within the scope of the following claims.

## Claims

1. A method of performing proteomic analysis comprising the steps of:
placing a single cell sample into a smaller volume nanowell disposed within a chip, the smaller volume nanowell configured to have a diameter < 2 mm and hold liquid volume < 1 µL;
placing a peptide mixture into a larger volume booster well, the larger volume booster well configured to have a diameter > 1 mm and hold liquid volume > 1 µL, wherein the diameter of the smaller volume nanowell is less than the diameter of the larger volume booster well;
lysing the cell sample;
extracting proteins from the cell sample;
digesting the proteins into peptides;
labeling the peptides and the peptide mixture using tandem mass tag (TMT) labels;
combining the labeled peptides from the nanowells and the booster wells into the booster well to form a mixed sample;
separating the mixed sample; and
acquiring data from the separated sample using a mass spectrometer, wherein the method further comprises the following steps:
the step of setting the automatic gain control levels on the mass spectrometer during MS2 or MS3 data collection > 5E5;
setting injection times during MS2 or MS3 data collection > 250 ms;
adjusting the automatic gain control levels and injection times based on the booster/sample ratios and the protein abundance in single cells; and
wherein separating the mixed sample includes a step of pre-fractionating the mixed sample into multiple fractions.

2. The method of claim 1 wherein the pre-fractionation step is performed on a nanoflow high PH liquid chromatography.

3. The method of claim 2 wherein the fraction is collected into a low volume container containing dilution buffer < 25 µL.

4. The method of claim 3, further comprising the following features:
viii. wherein the container is made from protein-low-binding material selected from the group consisting of polypropylene, polyethylene, epoxy, polyetheretherketone, and surface-modified glass; and
ix. wherein the dilution buffer contains at least one non-ionic and MS-compatible surfactants selected from the group consisting of n-Dodecyl β-D-maltoside, Triton X-100, Tween-20, Tween-80, and NP-40.

5. The method of claim 1 further comprising the step of washing the samples from the nanowell using a wash solution and combining the nanowell wash solution into the booster well prior to performing LC separation step.

6. The method of claim 1 further comprising the step of stabilizing the extracted peptides.

## Patentansprüche

1. Ein Verfahren zum Durchführen einer Proteomanalyse, das die folgenden Schritte beinhaltet:
Platzieren einer einzelnen Zellprobe in eine Nanovertiefung mit kleinerem Volumen, die innerhalb eines Chips angeordnet ist, wobei die Nanovertiefung mit kleinerem Volumen so konfiguriert ist, dass sie einen Durchmesser von < 2 mm aufweist und ein Flüssigkeitsvolumen von < 1 µL fasst;
Platzieren einer Peptidmischung in eine Boostervertiefung mit größerem Volumen, wobei die Boostervertiefung mit größerem Volumen so konfiguriert ist, dass sie einen Durchmesser von > 1 mm aufweist und ein Flüssigkeitsvolumen von > 1 µL fasst, wobei der Durchmesser der Nanovertiefung mit kleinerem Volumen kleiner ist als der Durchmesser der Boostervertiefung mit größerem Volumen;
Lysieren der Zellprobe;
Extrahieren von Proteinen aus der Zellprobe;
Aufschließen der Proteine in Peptide;
Markieren der Peptide und der Peptidmischung unter Verwendung von Tandem-Massen-Tag(TMT)-Markierungen;
Kombinieren der markierten Peptide von den Nanovertiefungen und den Boostervertiefungen in der Boostervertiefung, um eine gemischte Probe zu bilden;
Trennen der gemischten Probe; und
Erfassen von Daten von der getrennten Probe unter Verwendung eines Massenspektrometers, wobei das Verfahren ferner die folgenden Schritte beinhaltet:
den Schritt des Einstellens der automatischen Verstärkungssteuerungspegel auf dem Massenspektrometer während der MS2- oder MS3-Datenerfassung auf > 5E5;
Einstellen von Injektionszeiten während der MS2- oder MS3-Datenerfassung auf > 250 ms;
Einstellen der automatischen Verstärkungssteuerungspegel und der Injektionszeiten basierend auf den Booster/Probenverhältnissen und der Proteinhäufigkeit in einzelnen Zellen; und
wobei das Trennen der gemischten Probe einen Schritt des Vorfraktionierens der gemischten Probe in mehrere Fraktionen umfasst.

2. Verfahren gemäß Anspruch 1, wobei der Vorfraktionierungsschritt mit einer Nanoflow-Flüssigkeitschromatographie mit hohem pH-Wert durchgeführt wird.

3. Verfahren gemäß Anspruch 2, wobei die Fraktion in einem Behälter mit geringem Volumen gesammelt wird, der Verdünnungspuffer von < 25 µL enthält.

4. Verfahren gemäß Anspruch 3, das ferner die folgenden Merkmale beinhaltet:
viii. wobei der Behälter aus einem Material mit geringer Proteinbindung hergestellt ist, das aus der Gruppe ausgewählt ist, die aus Polypropylen, Polyethylen, Epoxid, Polyetheretherketon und oberflächenmodifiziertem Glas besteht; und
ix. wobei der Verdünnungspuffer mindestens ein nichtionisches und MS-kompatibles Tensid enthält, das aus der Gruppe ausgewählt ist, die aus n-Dodecyl-β-D-maltosid, Triton X-100, Tween-20, Tween-80 und NP-40 besteht.

5. Verfahren gemäß Anspruch 1, das ferner den Schritt des Waschens der Proben von der Nanovertiefung unter Verwendung einer Waschlösung und des Kombinierens der Nanovertiefungswaschlösung in die Boostervertiefung vor dem Durchführen des Trennschritts durch LC beinhaltet.

6. Verfahren gemäß Anspruch 1, das ferner den Schritt des Stabilisierens der extrahierten Peptide beinhaltet.

## Revendications

1. Une méthode de réalisation d'une analyse protéomique comprenant les étapes consistant :
à placer un échantillon de cellule unique dans un nanopuits de plus petit volume disposé au sein d'une puce, le nanopuits de plus petit volume étant configuré pour présenter un diamètre < 2 mm et contenir un volume de liquide < 1 µL ;
à placer un mélange de peptides dans un puits intensificateur (« booster well ») de plus grand volume, le puits intensificateur de plus grand volume étant configuré pour présenter un diamètre > 1 mm et contenir un volume de liquide > 1 µL, le diamètre du nanopuits de plus petit volume étant inférieur au diamètre du puits intensificateur de plus grand volume ;
à lyser l'échantillon de cellule ;
à extraire des protéines de l'échantillon de cellule ;
à digérer les protéines en peptides ;
à marquer les peptides et le mélange de peptides à l'aide de marqueurs à étiquettes de masse en tandem (TMT, « tandem mass tag ») ;
à combiner les peptides marqués provenant des nanopuits et des puits intensificateurs dans le puits intensificateur afin de former un échantillon mélangé ;
à séparer l'échantillon mélangé ; et
à acquérir des données à partir de l'échantillon séparé à l'aide d'un spectromètre de masse, la méthode comprenant en sus les étapes suivantes :
l'étape consistant à régler les niveaux de commande automatique de gain sur le spectromètre de masse durant le recueil de données par MS2 ou MS3 à > 5E5 ;
à régler des temps d'injection durant le recueil de données par MS2 ou MS3 à > 250 ms ;
à ajuster les niveaux de commande automatique de gain et les temps d'injection sur la base des rapports intensificateur/échantillon et de l'abondance de protéines dans des cellules uniques ; et
dans laquelle le fait de séparer l'échantillon mélangé inclut une étape consistant à préfractionner l'échantillon mélangé en de multiples fractions.

2. La méthode de la revendication 1 dans laquelle l'étape de préfractionnement est réalisée sur une chromatographie en phase liquide à pH élevé à nanoflux.

3. La méthode de la revendication 2 dans laquelle la fraction est recueillie dans un récipient à faible volume contenant du tampon de dilution à hauteur de < 25 µL.

4. La méthode de la revendication 3, comprenant en sus les caractéristiques suivantes :
viii. dans laquelle le récipient est fabriqué en un matériau à faible liaison aux protéines sélectionné dans le groupe constitué de polypropylène, polyéthylène, époxy, polyétheréthercétone, et verre à surface modifiée ; et
ix. dans laquelle le tampon de dilution contient au moins un tensioactif non ionique et compatible pour la MS sélectionné dans le groupe constitué de n-dodécyl β-D-maltoside, Triton X-100, Tween-20, Tween-80, et NP-40.

5. La méthode de la revendication 1 comprenant en sus l'étape consistant à laver les échantillons provenant du nanopuits à l'aide d'une solution de lavage et à combiner la solution de lavage de nanopuits dans le puits intensificateur avant de réaliser l'étape de séparation par LC.

6. La méthode de la revendication 1 comprenant en sus l'étape consistant à stabiliser les peptides extraits.
